# EUROPÄISCHE PATENTSCHRIFT

(11) **EP 2 324 037 B1**
(45) Veröffentlichungstag und Bekanntmachung des Hinweises auf die Patenterteilung: **19.09.2012**
(21) Anmeldenummer: 09781166.5
(22) Anmeldetag: 28.07.2009
(51) Int. Cl.: C07F 1/00, B01D 53/04, B01D 53/86

(54) **LITHIUMBASIERTE METALLORGANISCHE GERÜSTMATERIALIEN**
LITHIUM-BASED METAL ORGANIC STRUCTURAL MATERIALS
MATÉRIAUX SQUELETTES ORGANOMÉTALLIQUES À BASE DE LITHIUM

(30) Priorität: 30.07.2008 EP 08161478
(43) Veröffentlichungstag der Anmeldung: 25.05.2011
(73) Patentinhaber: BASF SE, 67056 Ludwigshafen (DE)
(72) Erfinder: LEUNG, Emi, 68161 Mannheim (DE); MÜLLER, Ulrich, 67435 Neustadt (DE); COX, Gerhard, 67098 Bad Dürkheim (DE)
(86) Internationale Anmeldenummer: PCT/EP2009/059716
(87) Internationale Veröffentlichungsnummer: WO 2010/012715

(56) Entgegenhaltungen:
- US-A1- 2005 154 222

## Beschreibung

Die vorliegende Erfindung betrifft ein poröses metallorganisches Gerüstmaterial, dessen Herstellung sowie Verfahren zur Speicherung oder Abtrennung eines Gases mit Hilfe des Gerüstmaterials und dessen entsprechender Verwendung.

Materialien zur Speicherung oder Trennung von Gasen sind im Stand der Technik bekannt. Beispielsweise sind Aktivkohle und Molekularsiebe sowie metallorganische Gerüstmaterialien zu nennen.

Hierbei zeichnen sich die letztgenannten metallorganischen Gerüstmaterialien besonders dadurch aus, dass durch entsprechende Wahl des Metalls sowie des Liganden Speicher- oder Trennmaterialien erhalten werden können, die für spezielle Anwendungen eingesetzt werden können.

Darüber hinaus besteht insbesondere ein Bedarf an kostengünstigen und robusten Materialien, die sich insbesondere durch ein selektives Verhalten bei der Speicherung oder Trennung gegenüber bestimmten Gasen auszeichnen.

Es hat sich bei metallorganischen Gerüstmaterialien herausgestellt, dass durch geeignete Synthese Gerüstmaterialien erhalten werden können, deren Bestandteile aus konventionellen Salzen bekannt sind.

So ist beispielsweise ein poröses metallorganisches Gerüstmaterial, welches Kanäle aufweist, von A. Lavalette et al., Eur. J. Inorg. Chem. 2004, 3981-3983, bekannt, welches ein gemischtes Natriumacetat und Perchlorat darstellt. Hierbei wird jedoch betont, dass für die Herstellung eines metallorganischen Gerüstmaterials ein Templat erforderlich ist, so dass ohne ein solches die reine Mischung von Natriumacetat und -perchlorat lediglich die Bildung des hydratisierten Natriumacetats oder Natriumperchlorats ergibt.

Auch für Natriumacetat wird eine zweidimensionale Schicht von L.-Y. Hsu et al., Acta Crystallogr., Sect. C 1983, 39, 690-694, beschrieben, wobei sich das Gerüst dadurch ausbildet, dass Natriumionen sechsfach koordiniert an ein zweizähnig bindendes Acetat und vier einzähnig bindende Acetatgruppen koordiniert.

US 2005/0154222 A1 beschreibt die Herstellung von metallorganischen Gerüstmaterialien, wobei keine Lithium-haltigen Materialien offenbart werden.

Trotz der im Stand der Technik bekannten metallorganischen Gerüstmaterialien besteht nach wie vor ein Bedarf an metallorganischen Gerüstmaterialien, die sich insbesondere durch ihr Verhalten bei der Speicherung oder Trennung gegenüber Gasen auszeichnen.

Eine Aufgabe der vorliegenden Erfindung besteht somit darin, geeignete Gerüstmaterialien bereitzustellen.

Die Aufgabe wird gelöst durch ein poröses metallorganisches Gerüstmaterial enthaltend mindestens eine erste organische Verbindung und Ionen mindestens eines Metalls, wobei das Gerüst des Gerüstmaterials zumindest teilweise dadurch gebildet wird, dass die mindestens erste organische Verbindung zumindest teilweise zweizähnig an mindestens zwei Ionen des mindestens einen Metalls koordinativ bindet, wobei das mindestens eine Metall Lithium ist und wobei sich die mindestens erste Verbindung von Ameisensäure oder Essigsäure ableitet.

Es hat sich gezeigt, dass poröse metallorganische Gerüstmaterialien wie oben beschrieben erhalten werden können, die auf Grund ihrer kleinen spezifischen Oberfläche und Porenstruktur geeignet für die Gasadsorption und -trennung und insbesondere für die Speicherung und selektive Trennung atomar oder molekular kleiner Gase geeignet sind.

Im Rahmen der vorliegenden Erfindung ist unter dem Begriff "ableiten" zu verstehen, dass Ameisensäure und/oder Essigsäure im porösen metallorganischen Gerüstmaterial gemäß der vorliegenden Erfindung als Formiat bzw. Acetat vorliegen, wobei auch teilweise eine protonierte Form möglich ist.

Das erfindungsgemäße poröse metallorganische Gerüstmaterial zeichnet sich also dadurch aus, dass dessen Gerüst zumindest teilweise von Ionen mindestens eines Metalls und mindestens einer ersten organischen Verbindung aufgebaut wird, wobei die mindestens erste organische Verbindung zumindest teilweise an zwei unterschiedliche Metallionen bindet, so dass eine verbrückende Struktur zum Gerüstaufbau ermöglicht ist. Die beiden Ionen des mindestens einen Metalls können somit Ionen eines Metalls oder verschiedener Metalle sein, sofern das Gerüstmaterial mehrere verschiedene Metalle aufweist.

Hierbei müssen jedoch zumindest Lithiumionen vorhanden sein. Bevorzugt sind keine weiteren Metallionen außer Lithiumionen am Gerüstaufbau beteiligt.

Die mindestens erste organische Verbindung kann sich von Ameisensäure oder Essigsäure ableiten.

Das erfindungsgemäße poröse metallorganische Gerüstmaterial kann somit formiatbasiert oder acetatbasiert sein.

Es ist jedoch auch möglich, dass neben der ersten organischen Verbindung eine weitere organische Verbindung vorhanden ist, wobei sich die mindestens erste organische Verbindung von Ameisensäure und die mindestens zweite organische Verbindung von Essigsäure ableiten. Das erfindungsgemäße poröse metallorganische Gerüstmaterial basiert dann auf einem gemischten Gerüst Formiat und Acetat enthaltend.

Es ist insbesondere bevorzugt, dass in dem erfindungsgemäßen porösen metallorganischen Gerüstmaterial neben Ameisensäure keine weitere organische Verbindung am Gerüstaufbau beteiligt ist.

Weiterhin besonders bevorzugt ist ein poröses metallorganisches Gerüstmaterial, das neben Essigsäure keine weitere organische Verbindung aufweist.

Schließlich ist ebenfalls bevorzugt, dass neben Ameisensäure und Essigsäure keine weitere organische Verbindung am Gerüstaufbau beteiligt ist.

Es sind insbesondere erfindungsgemäße poröse Gerüstmaterialien bevorzugt, bei denen das Gerüst ausschließlich aus Li⁺ und Formiat, Li⁺ und Acetat oder Li⁺, Formiat und Acetat aufgebaut ist.

Sofern sowohl eine erste als auch eine zweite organische Verbindung im erfindungsgemäßen Gerüstmaterial vorliegen, können verschiedene molare Verhältnisse von erster und zweiter organischer Verbindung auftreten.

Vorzugsweise liegt das molare Verhältnis von erster zu zweiter organischer Verbindung im erfindungsgemäßen metallorganischen Gerüstmaterial im Bereich von 10 : 1 bis 1 : 10. Mehr bevorzugt liegt das Verhältnis im Bereich von 5 : 1 bis 1 : 5, weiter mehr bevorzugt im Bereich von 2 : 1 bis 1 : 2, weiterhin mehr bevorzugt im Bereich von 1,5 : 1 bis 1 : 1,5, weiter mehr bevorzugt im Bereich von 1,2 :1 bis 1 : 1,2, weiter mehr bevorzugt im Bereich von 1,1 : 1 bis 1 : 1,1 und insbesondere bei 1 : 1. Entsprechend können die bei der Herstellung erforderlichen Mengen an Ameisensäure und Essigsäure eingesetzt werden.

Ein weiterer Gegenstand der vorliegenden Erfindung ist ein Verfahren zur Herstellung eines erfindungsgemäßen porösen metallorganischen Gerüstmaterials, die Schritte enthaltend
(a) Umsetzen einer Reaktionslösung enthaltend Lithiumnitrat, Ameisensäure und/oder Essigsäure sowie einem Lösemittel bei einer Temperatur im Bereich von 110°C bis 150°C für mindestens einen Tag und
(b) Abtrennen des ausgefallenen Feststoffes.

Das erfindungsgemäße Verfahren zur Herstellung des erfindungsgemäßen Gerüstmaterials enthält als Schritt (a) das Umsetzen einer Reaktionslösung, enthaltend Lithiumnitrat, Ameisensäure und/oder Essigsäure sowie einem Lösemittel bei einer Temperatur im Bereich von 110°C bis 150°C für mindestens einen Tag.

Bevorzugt erfolgt die Umsetzung zumindest zeitweise, insbesondere zu Beginn der Umsetzung, unter Rühren. Dies gilt insbesondere dann, wenn größere Mengen umgesetzt werden sollen.

Als eine Ausgangsverbindung wird Lithiumnitrat verwendet. Vorzugsweise liegt dessen Anfangskonzentration in der Reaktionslösung im Bereich von 0,01 mol/l bis 10 mol/l. Weiterhin bevorzugt liegt die Anfangskonzentration im Bereich von 0,1 mol/l bis 5 mol/l. Weiterhin bevorzugt liegt die Anfangskonzentration im Bereich von 0,2 mol/l bis 2,5 mol/l. Insbesondere liegt die Anfangskonzentration im Bereich von 0,3 mol/l bis 1 mol/l.

Die Menge an Lithiumnitrat wird dabei in einer Menge der Reaktionslösung zugeführt, so dass auf Grund des ausgefallenen Feststoffes in Schritt (a) die Lithiumkonzentration in der Reaktionslösung abnimmt.

Weiterhin ist es bevorzugt, dass das Verhältnis der anfänglichen Stoffmenge an eingesetzter Ameisensäure bzw. eingesetzter Essigsäure zur anfänglichen Stoffmenge an Lithiumnitrat im Bereich von 1:1 bis 3:1 liegt. Weiter bevorzugt liegt das Verhältnis im Bereich von 1,25:2, weiter bevorzugt im Bereich von 1,4:1 bis 1,6:1. Sofern Ameisensäure und Essigsäure vorhanden sind, muss entsprechend die Summe der anfänglichen Stoffmengen von Ameisensäure und Essigsäure berücksichtigt werden.

Die Reaktionslösung für Schritt (a) des erfindungsgemäßen Verfahrens zur Herstellung des erfindungsgemäßen metallorganischen Gerüstmaterials enthält neben Lithiumnitrat und Ameisensäure und/oder Essigsäure weiterhin ein Lösemittel.

Das Lösemittel sollte geeignet sein, die eingesetzten Ausgangsstoffe zumindest teilweise in Lösung zu bringen. Darüber hinaus sollte das Lösemittel derart gewählt werden, dass der erforderliche Temperaturbereich eingehalten werden kann.

Die Umsetzung in dem erfindungsgemäßen Verfahren zur Herstellung des erfindungsgemäßen Materials erfolgt somit in Gegenwart eines Lösemittels. Hierbei können Solvothermalbedingungen eingesetzt werden. Unter dem Begriff "thermal" ist im Rahmen der vorliegenden Erfindung ein Herstellverfahren zu verstehen, bei dem die Umsetzung in einem Druckbehälter derart durchgeführt wird, dass dieser während der Umsetzung verschlossen ist und erhöhte Temperatur angelegt wird, so dass aufgrund des Dampfdruckes von vorhandenem Lösemittel sich ein Druck innerhalb des Reaktionsmediums im Druckbehälter aufbaut. Hierdurch kann die gewünscht Umsetzungstemperatur gegebenenfalls erreicht werden.

Vorzugsweise erfolgt die Umsetzung nicht in Wasser enthaltendem Medium. Die Umsetzung in dem erfindungsgemäßen Verfahren erfolgt demzufolge vorzugsweise in Gegenwart eines nicht-wässrigen Lösemittels.

Die Umsetzung kann beispielsweise bei einem Druck von höchstens 2 bar (absolut) erfolgen. Sofern die Umsetzung nicht unter Solvothermalbedingungen erfolgt, beträgt der Druck vorzugsweise höchstens 1230 mbar (absolut). Insbesondere bevorzugt findet dann die Umsetzung bei Atmosphärendruck statt. Hierbei kann es jedoch apparativ bedingt zu leichten Über- oder Unterdrücken kommen. Daher ist im Rahmen der vorliegenden Erfindung unter dem Begriff "Atmosphärendruck" derjenige Druckbereich zu verstehen, der sich aus dem tatsächlichen vorliegenden Atmosphärendruck ± 150 mbar ergibt.

Die Umsetzung findet in einem Temperaturbereich von 110 °C bis 150 °C statt. Vorzugsweise liegt die Temperatur im Bereich von 115 °C bis 130 °C. Weiterhin bevorzugt liegt die Temperatur in einem Bereich von 120 °C bis 125 °C.

Die Reaktionslösung kann weiterhin eine Base aufweisen. Durch die Verwendung eines organischen Lösemittels ist es häufig nicht erforderlich, eine solche Base einzusetzen. Nichts desto trotz kann das Lösemittel für das erfindungsgemäße Verfahren derart gewählt werden, dass dieses als solches basisch reagiert, was jedoch nicht zwingend für die Durchführung des erfindungsgemäßen Verfahrens sein muss.

Ebenso kann eine Base eingesetzt werden. Bevorzugt ist jedoch, dass keine zusätzliche Base eingesetzt wird.

Es ist weiterhin vorteilhaft, dass die Umsetzung unter Rühren stattfinden kann, was auch bei einem Scale-up vorteilhaft ist.

Das (nicht-wässrige) organische Lösemittel ist vorzugsweise ein C₁₋₆-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogeniertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Bevorzugte Lösemittel sind THF, DMF, DEF, DMAc und NMP. Besonders bevorzugt sind THF und DMF.

Vorzugsweise enthält das Lösemittel die oben genannten Substanzen. Weiter bevorzugt werden die oben genannten Lösemittel in reiner Form, also nicht als Gemische eingesetzt.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Schritt (a) des erfindungsgemäßen Verfahrens zur Herstellung des erfindungsgemäßen Gerüstmaterials wird für mindestens einen Tag durchgeführt. Vorzugsweise erfolgt die Umsetzung mindestens 1,5 Tage, weiter bevorzugt mindestens 2 Tage, weiter bevorzugt mindestens 2,5 Tage, weiter bevorzugt mindestens 3 Tage.

Weiterhin weist das erfindungsgemäße Verfahren den Schritt (b), Abtrennen des ausgefallenen Feststoffes, auf.

Auf Grund von Schritt (a) des erfindungsgemäßen Herstellverfahrens fällt das Gerüstmaterial als Feststoff aus der Reaktionslösung aus. Eine Abtrennung erfolgt durch im Stand der Technik bekannte Methoden, wie Filtration oder dergleichen.

Das erfindungsgemäße poröse metallorganische Gerüstmaterial eignet sich zur Speicherung oder Abtrennung eines Gases.

Dementsprechend ist ein weiterer Gegenstand der vorliegenden Erfindung ein Verfahren zur Speicherung oder Abtrennung eines Gases den Schritt enthaltend
- In-Kontakt-Bringen des Gases oder eines das Gas enthaltenden Gasgemisches mit einem erfindungsgemäßen porösen metallorganischen Gerüstmaterial.

Dementsprechend ist weiterhin ein weiterer Gegenstand der vorliegenden Erfindung die Verwendung eines erfindungsgemäßen porösen metallorganischen Gerüstmaterials zur Speicherung oder Abtrennung eines Gases.

Die Gasadsorption bzw. -trennung erfolgt grundsätzlich nach dem Stand der Technik bekannten Verfahren.

Grundlagen und technische Verfahren sind beispielsweise in Werner Kast, Adsorption aus der Gasphase, VCH Weinheim, 1988, beschrieben.

Die Druckwechseladsorption ist beispielsweise in D. M. Ruthwen et al., Wiley-VCH, 1993, beschrieben.

Bei dem zu adsorbierenden oder abzutrennenden Gas kann es sich beispielsweise um Wasserstoff, Erdgas, Stadtgas, Kohlenwasserstoffe, insbesondere Methan, Ethan, Ethin, Ethen, Propan, N-Butan sowie I-Butan, Kohlenmonoxid, Kohlendioxid, Stickoxide, Sauerstoff, Schwefeloxide, Halogene, halogenierte Kohlenwasserstoffe, NF₃, SF₆, Ammoniak, Borane, Phosphane, Schwefelwasserstoff, Amine, Formaldehyd, Edelgase, insbesondere Helium, Neon, Argon, Krypton sowie Xenon, Stickstoff oder Sauerstoff oder ein Gemisch davon handeln.

Bevorzugt ist das Gas ausgewählt aus der Gruppe bestehend aus Stickstoff, Wasserstoff, Sauerstoff und Methan.

### Beispiele

### Beispiel 1 Li-Formiat metallorganisches Gerüstmaterial aus THF

| | | | |
|---|---|---|---|
| Einsatzstoffe: | 5,3g | LiNO₃ | = 76,9 mmol |
| | 5,3g | Ameisensäure | =115,1 mmol |
| Lösemittel: | 140,0g | THF | = 1,94 mol |

Es wird das Lithiumnitrat in THF in einem Autoklavenbecher aufgelöst. Dazu gibt man die Ameisensäure und rührt die Lösung für 10 min.

### Kristallisation:

125 °C / 78h / statische Bedingungen

### Aufarbeitung:

Nach Abkühlen werden die entstandenen Kristalle abfiltriert und der Filterkuchen wird 3-mal mit 50 ml DMF gewaschen.

Ausbeute: 3,2 g

### Analytik:

- Oberfläche: 20 m²/g nach Langmuir (N₂)

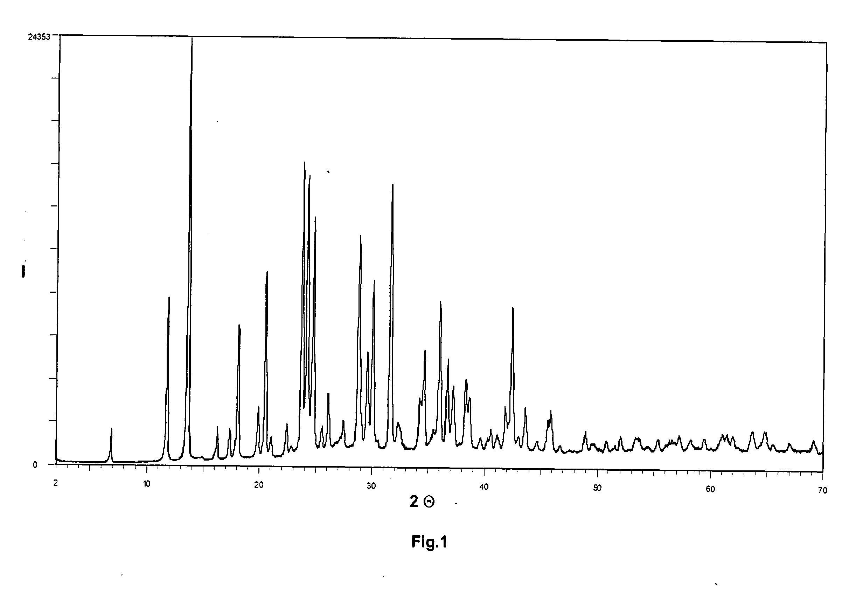

Fig. 1 zeigt das Röntgendiffraktogramm des erfindungsgemäßen metallorganischen Gerüstmaterials aus Li-Formiat. Hierbei beschreiben in dem Diffraktogramm I die Intensität (Lᵢₙ (Counts)) und 2 ⊝ die Theta Skala.

### Beispiel 2 Li-Formiat metallorganisches Gerüstmaterial aus DMF

| | | | |
|---|---|---|---|
| Einsatzstoffe: | 51,6 g | LiNO₃ | = 748,4 mmol |
| | 51,2 g | Ameisensäure | = 1112,32 mmol |
| Lösemittel: | 516 g | DMF | = 7,1 mol |

### Versuchsdurchführung

a) Synthese: Lithiumnitrat wird im 1 I-Vierhalskolben unter N₂ in DMF gelöst (exotherm bis 40°C). Anschließend wird vorsichtig Ameisensäure zugegeben (exotherm bis 41°C), 1h bei Raumtemperatur (RT) gerührt, danach im Ölbad auf 125°C erwärmt und 120 h bei 125°C unter N₂ gerührt.
b) Aufarbeitung: Nach Abkühlen auf Raumtemperatur wird das Reaktionsgemisch in 1,5 I Chloroform gegeben, auf < 10°C abgekühlt und unter N₂ abfiltriert.
c) Trocknung: Das erhaltene metallorganische Gerüstmaterial wird 0,5 h mit Hilfe einer Glasfrittennutsche unter N₂ trockengesaugt, 2 h bei 60°C und danach 8 h bei
130°C und 50 mbar im Vakuumtrockenschrank getrocknet.

Farbe: hellrosa; Ausbeute: 6,8 g

### Analytik

- Oberfläche: 2,31 m²/g nach Langmuir (N₂)
- E. Analyse: C 21,9%; H 2,1%, O 63%; N 1%; Li 12,7%

### Beispiel 3 Li-Acetat metallorganisches Gerüstmaterial aus DMF

| | | | |
|---|---|---|---|
| Einsatzstoffe: | 51,6 g | LiNO₃ | = 748,4 mmol |
| | 66,8 g | Essigsäure | = 1112,32 mmol |
| Lösemittel: | 516 g | DMF | = 7,1 mol |

### Versuchsdurchführung

Die Synthese, Aufarbeitung und Trocknung erfolgt wie in Beispiel 2.

Farbe: farblos; Ausbeute: 5,2 g

### Analytik:

- Oberfläche: 0,175 m²/g nach Langmuir (N₂)
- E. Analyse: C 25,2%; H 2,7%; O 57%; N 1,2%, Li 11,9%

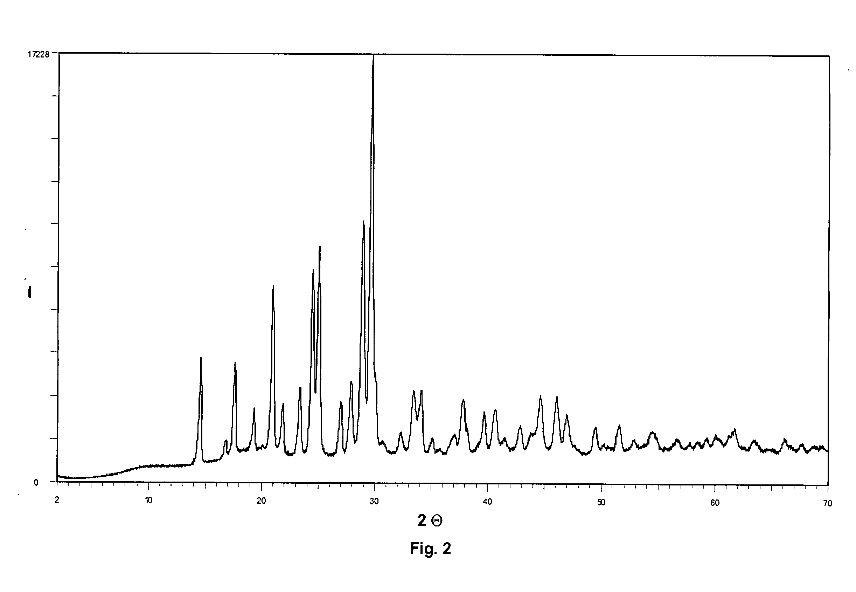

Fig. 2 zeigt das Röntgendiffraktogramm des erfindungsgemäßen metallorganischen Gerüstmaterials aus Li-Acetat. Hierbei beschreiben in dem Diffraktogramm I die Intensität (Lᵢₙ (Counts)) und 2 ⊝ die Theta Skala.

## Patentansprüche

1. Poröses metallorganisches Gerüstmaterial enthaltend mindestens eine erste organische Verbindung und Ionen mindestens eines Metalls, wobei das Gerüst des Gerüstmaterials zumindest teilweise dadurch gebildet wird, dass die mindestens erste organische Verbindung zumindest teilweise zweizähnig an mindestens zwei Ionen des mindestens einen Metalls koordinativ bindet, wobei das mindestens eine Metall Lithium ist und wobei sich die mindestens erste Verbindung von Ameisensäure oder Essigsäure ableitet.

2. Gerüstmaterial nach Anspruch 1, **dadurch gekennzeichnet, dass** neben der mindestens ersten Verbindung mindestens eine zweite Verbindung zumindest teilweise zweizähnig an die Ionen des mindestens einen Metalls koordinativ bindet.

3. Gerüstmaterial nach Anspruch 2, **dadurch gekennzeichnet, dass** sich die mindestens erste organische Verbindung von Ameisensäure und die mindestens zweite organische Verbindung von Essigsäure ableitet.

4. Gerüstmaterial nach einem der Ansprüche 1 bis 3, **dadurch gekennzeichnet, dass** nur eine oder zwei organische Verbindungen und keine weiteren organischen Verbindungen das Gerüst aufbauen.

5. Gerüstmaterial nach einem der Ansprüche 1 bis 4, **dadurch gekennzeichnet, dass** nur Lithiumionen und keine weiteren Ionen eines Metalls das Gerüst aufbauen.

6. Verfahren zur Herstellung eines porösen metallorganischen Gerüstmaterials nach einem der Ansprüche 1 bis 5, die Schritte enthaltend
(a) Umsetzen einer Reaktionslösung enthaltend Lithiumnitrat, Ameisensäure und/oder Essigsäure sowie einem Lösemittel bei einer Temperatur im Bereich von 110°C bis 150°C für mindestens einen Tag und
(b) Abtrennen des ausgefallenen Feststoffes.

7. Verfahren nach Anspruch 6, **dadurch gekennzeichnet, dass** das Lösemittel N,N-Dimethylformamid oder Tetrahydrofuran enthält.

8. Verfahren zur Speicherung oder Abtrennung eines Gases den Schritt enthaltend
• In Kontakt bringen des Gases oder eines das Gas enthaltenden Gasgemisches mit einem porösen metallorganischen Gerüstmaterials nach einem der Ansprüche 1 bis 5.

9. Verfahren nach Anspruch 8, **dadurch gekennzeichnet, dass** das Gas ausgewählt ist aus der Gruppe bestehend aus Stickstoff, Wasserstoff, Sauerstoff und Methan.

10. Verwendung eines porösen metallorganischen Gerüstmaterials nach einem der Ansprüche 1 bis 5 zur Speicherung oder Abtrennung eines Gases.

## Claims

1. A porous metal organic framework comprising at least one first organic compound and ions of at least one metal, with the skeleton of the framework being formed at least partly by the at least one first organic compound coordinating at least partly in a bidentate fashion to at least two ions of the at least one metal, where the at least one metal is lithium and the at least one first compound is derived from formic acid or acetic acid.

2. The framework according to claim 1, wherein, in addition to the at least one first compound, at least one second compound coordinates at least partly in a bidentate fashion to the ions of the at least one metal.

3. The framework according to claim 2, wherein the at least one first organic compound is derived from formic acid and the at least one second organic compound is derived from acetic acid.

4. The framework according to any of claims 1 to 3, wherein only one or two organic compounds and no further organic compounds make up the framework.

5. The framework according to any of claims 1 to 4, wherein only lithium ions and no further ions of a metal make up the framework.

6. A process for preparing a porous metal organic framework according to any of claims 1 to 5, which comprises the steps
(a) reaction of a reaction solution comprising lithium nitrate, formic acid and/or acetic acid and also a solvent at a temperature in the range from 110°C to 150°C for at least one day and
(b)isolation of the precipitated solid.

7. The process according to claim 6, wherein the solvent comprises N,N-dimethylformamide or tetrahydrofuran.

8. A process for storing or separating off a gas, which comprises the step
• contacting of the gas or a gas mixture comprising the gas with a porous metal organic framework according to any of claims 1 to 5.

9. The process according to claim 8, wherein the gas is selected from the group consisting of nitrogen, hydrogen, oxygen and methane.

10. The use of a porous metal organic framework according to any of claims 1 to 5 for storing or separating off a gas.

## Revendications

1. Matériau d'ossature organométallique poreux contenant au moins un premier composé organique et des ions d'au moins un métal, l'ossature du matériau d'ossature étant formée au moins en partie par le fait que ledit au moins premier composé organique se lie par coordination au moins en partie en mode bidenté à au moins deux ions dudit au moins un métal, ledit au moins un métal étant le lithium et ledit au moins premier composé dérivant de l'acide formique ou de l'acide acétique.

2. Matériau d'ossature selon la revendication 1, **caractérisé en ce qu'**outre ledit au moins premier composé au moins un deuxième composé se lie par coordination au moins en partie en mode bidenté aux ions dudit au moins un métal.

3. Matériau d'ossature selon la revendication 2, **caractérisé en ce que** ledit au moins premier composé organique dérive de l'acide formique et ledit au moins deuxième composé organique dérive de l'acide acétique.

4. Matériau d'ossature selon l'une quelconque des revendications 1 à 3, **caractérisé en ce que** seulement un ou deux composés organiques et à l'exclusion d'autres composés organiques constituent l'ossature.

5. Matériau d'ossature selon l'une quelconque des revendications 1 à 4, **caractérisé en ce que** seuls des ions lithium et à l'exclusion d'autres ions d'un métal constituent l'ossature.

6. Procédé pour la préparation d'un matériau d'ossature organométallique poreux selon l'une quelconque des revendications 1 à 5, comportant les étapes
(a) mise en réaction d'une solution réactionnelle contenant du nitrate de lithium, de l'acide formique et/ou de l'acide acétique ainsi qu'un solvant, à une température dans la plage de 110°C à 150 °C pendant au moins un jour et
(b) séparation du solide formé.

7. Procédé selon la revendication 6, **caractérisé en ce que** le solvant contient du N,N-diméthylformamide ou du tétrahydrofurane.

8. Procédé pour le stockage ou la séparation d'un gaz, comportant l'étape
• mise en contact du gaz ou d'un mélange gazeux contenant le gaz, avec un matériau d'ossature organométallique poreux selon l'une quelconque des revendications 1 à 5.

9. Procédé selon la revendication 8, **caractérisé en ce que** le gaz est choisi dans le groupe consistant en azote, hydrogène, oxygène et méthane.

10. Utilisation d'un matériau d'ossature organométallique poreux selon l'une quelconque des revendications 1 à 5, pour le stockage ou la séparation d'un gaz.
